# EUROPEAN PATENT APPLICATION

(11) **EP 4 575 851 A1**
(43) Date of publication of application: **25.06.2025**
(21) Application number: 24222182.8
(22) Date of filing: 20.12.2024
(51) Int. Cl.: G06F 21/44, G06F 21/56, G06F 21/85, G16H 40/63

(54) **EXTERNAL DEVICE AUTHENTICATION**

(30) Priority: 21.12.2023 US 202363613707 P
(71) Applicant: Physio-Control, Inc., Redmond, WA 98052 (US)
(72) Inventor: PADMANABHA, Seshadri K., Redmond, 98052 (US); SKELTON, Dennis M., Redmond, 98052 (US); DUKE, Steven Barry, Redmond, 98052 (US); RUTZER, Mark, Redmond, 98052 (US); MACKIE, Richard, Redmond, 98052 (US); WOOTTEN, James, Redmond, 98052 (US); KILLEBREW, Mark G., Redmond, 98052 (US); FROLOV, Alexander, Redmond, 98052 (US); STEWART, David B., Redmond, 98052 (US); BALES, Robert, Redmond, 98052 (US); BEUNING, Gail R., Redmond, 98052 (US)
(74) Representative: V.O.

(57) **Abstract**

A method includes performing, by a device, one or more device-specific tasks while operating in a first mode of operation. The method includes detecting an insertion of an external storage device into an external storage device interface of the device. The method includes performing a first authentication check on the external storage device. The method includes transitioning from the first mode of operation to a second mode of operation in response to the external storage device passing the first authentication check. The one or more device-specific tasks are suspended during the second mode of operation. The method includes performing a second authentication check on content stored on the external storage device during the second mode of operation. The method includes mounting the content stored on the external storage device in response to the content stored on the external storage device passing the second authentication check.

## Description

### Field

The present disclosure generally relates to device authentication, and more particularly, to authenticating an external storage device prior to mounting the external storage device.

### Background

A device can be configured to mount an external storage device, such as a thumb drive or a universal serial bus (USB) drive, when the external storage device is inserted into a corresponding interface of the device. To illustrate, the device can include an external storage device interface, such as a USB port, that is configured to receive the external storage device. Upon inserting (e.g., plugging in) the external storage device into the external storage device interface, content stored on the external storage device can be accessible by the device. For example, the device can mount the external storage device and access the content stored on the external storage device. However, in some scenarios, external storage devices can include malware (e.g., malicious software) that can cause the device to malfunction.

### Summary

In one aspect, the present application discloses a device. The device includes an external storage device interface, a processor, and a memory storing computer-executable instructions that, when executed by the processor, cause the processor to perform one or more device-specific tasks while operating in a first mode of operation. The computer-executable instructions also cause the processor to detect an insertion of an external storage device into the external storage device interface. The computer-executable instructions also cause the processor to perform a first authentication check on the external storage device. The computer-executable instructions also cause the processor to transition from the first mode of operation to a second mode of operation in response to the external storage device passing the first authentication check. The one or more device-specific tasks are suspended during the second mode of operation. The computer-executable instructions also cause the processor to perform a second authentication check on content stored on the external storage device during the second mode of operation. The computer-executable instructions also cause the processor to mount the content stored on the external storage device in response to the content stored on the external storage device passing the second authentication check.

In another aspect, the present application discloses a method. The method includes performing, by a device, one or more device-specific tasks while operating in a first mode of operation. The method also includes detecting, by the device, an insertion of an external storage device into an external storage device interface of the device. The method also includes performing, by the device, a first authentication check on the external storage device. The method also includes transitioning from the first mode of operation to a second mode of operation in response to the external storage device passing the first authentication check. The one or more device-specific tasks are suspended during the second mode of operation. The method also includes performing, by the device, a second authentication check on content stored on the external storage device during the second mode of operation. The method also includes mounting, by the device, the content stored on the external storage device in response to the content stored on the external storage device passing the second authentication check.

In another aspect, the present application discloses a non-transitory computer-readable medium. The non-transitory computer-readable medium includes instructions that, when executed by a processor of a device, causes the processor to perform operations. The operations include performing one or more device-specific tasks while operating in a first mode of operation. The operations also include detecting an insertion of an external storage device into an external storage device interface of the device. The operations also include performing a first authentication check on the external storage device. The operations also include transitioning from the first mode of operation to a second mode of operation in response to the external storage device passing the first authentication check. The one or more device-specific tasks are suspended during the second mode of operation. The operations also includes performing a second authentication check on content stored on the external storage device during the second mode of operation. The operations also include mounting the content stored on the external storage device in response to the content stored on the external storage device passing the second authentication check.

In another aspect, the present application discloses a device. The device includes an accessory device interface, a processor, and a memory storing computer-executable instructions that, when executed by the processor, cause the processor to perform one or more device-specific tasks while operating in a first mode of operation. The computer-executable instructions also cause the processor to detect an insertion of an accessory device into the accessory device interface. The computer-executable instructions also cause the processor to transition from the first mode of operation to a second mode of operation in response to the detecting insertion of the accessory device into the accessory device interface. The one or more device-specific tasks are suspended during the second mode of operation. The computer-executable instructions also cause the processor to perform, during the second mode of operation, an accessory device authentication check on the accessory device based on information from a remote server.

In another aspect, the present application discloses a method. The method includes performing, by a device, one or more device-specific tasks while operating in a first mode of operation. The method also includes detecting, by the device, an insertion of an accessory device into the accessory device interface. The method also includes transitioning from the first mode of operation to a second mode of operation in response to the detecting insertion of the accessory device into the accessory device interface. The one or more device-specific tasks are suspended during the second mode of operation. Alternatively, some, but not all of the one or more device-specific tasks are suspended during the second mode of operation. The method also includes performing, during the second mode of operation, an accessory device authentication check on the accessory device based on information from a remote server.

In another aspect, the present application discloses a non-transitory computer-readable medium. The non-transitory computer-readable medium includes instructions that, when executed by a processor of a device, causes the processor to perform operations. The operations include performing one or more device-specific tasks while operating in a first mode of operation. The operations also include detecting an insertion of an accessory device into the accessory device interface. The operations also include transitioning from the first mode of operation to a second mode of operation in response to the detecting insertion of the accessory device into the accessory device interface. The one or more device-specific tasks are suspended during the second mode of operation. Alternatively, some, but not all of the one or more device-specific tasks are suspended during the second mode of operation. The operations also include performing, during the second mode of operation, an accessory device authentication check on the accessory device based on information from a remote server.

The foregoing summary is illustrative only and is not intended to be in any way limiting. In addition to the illustrative aspects, embodiments, and features described above, further aspects, embodiments, and features will become apparent by reference to the figures and the following detailed description.

### Brief Description of the Drawings

A more complete understanding of embodiments of the present application may be derived by referring to the detailed description and claims when considered in conjunction with the following figures, wherein like reference numbers may refer to similar elements throughout the figures. The figures are provided to facilitate understanding of the disclosure without limiting the breadth, scope, scale, or applicability of the disclosure. The drawings are not necessarily made to scale.
Figure 1 illustrates a system that is operable to authenticate external devices inserted into a medical device, according to an exemplary embodiment;
Figure 2 illustrates different examples of medical devices, external storage devices, and accessory devices, according to an exemplary embodiment;
Figure 3 illustrates a process for authenticating an external storage device inserted into a medical device, according to an exemplary embodiment;
Figure 4 illustrates a process for authenticating an accessory device inserted into a medical device, according to an exemplary embodiment;
Figure 5 is a flowchart of a method for authenticating external devices inserted into a medical device, according to an exemplary embodiment; and
Figure 6 is a flowchart of another method for authenticating external devices inserted into a medical device, according to an exemplary embodiment.

### Detailed Description

The following description describes techniques for authenticating external devices. A medical device can be configured to mount an external storage device, such as a thumb drive or a universal serial bus (USB) drive, when the external storage device is inserted into a corresponding interface of the medical device. To illustrate, the medical device (e.g., a defibrillator, a patient monitoring device, etc.) can include an external storage device interface, such as a USB port, that is configured to receive the external storage device. Upon inserting (e.g., plugging in) the external storage device into the external storage device interface, content stored on the external storage device can be accessible by the medical device. For example, the medical device can mount the external storage device and access the content stored on the external storage device. However, in some scenarios, external storage devices can include malware (e.g., malicious software) that can cause the medical device to malfunction. The risk of malware affecting the operation of life-saving medical devices raises a particular concern. To reduce the risk associated with mounting the external storage device to the medical device, the techniques described herein provide a multi-step authentication process for mounting the external storage device.

Prior to inserting the external storage device into the external storage device interface of the medical device, the medical device can operate in a first mode of operation (e.g., a normal operation mode). While operating in the first mode of operation, the medical device can perform device-specific tasks. As a non-limiting example, if the medical device is a defibrillator, while operating in the first mode of operation, the medical device can perform defibrillation operations (e.g., apply an electric charge or current to a heart to restore a normal heartbeat). As another non-limiting example, if the medical device is a patient monitoring device, the medical device can monitor and record patient vital signs. Further, during this first mode of operation data ports, such as USB ports, may be disabled by the medical device software.

When the medical device detects that the external storage device has been inserted (e.g., plugged in), the medical device can perform a first authentication check on the external storage device prior to mounting the external storage device. In this manner, the medical device can block out incompatible, tampered or counterfeit accessories. During the first authentication check, the medical device can verify an identity of the external storage device using different attributes of the external storage device. As non-limiting examples, the medical device can verify the identity of the external storage device using a product identifier of the external storage device, a vendor identifier of the external storage device, or both.

If the external storage device does not pass the first authentication check, to reduce the likelihood of malfunction, content on the external storage device is not mounted and the external storage device is removed. However, if the external storage device passes the first authentication check (e.g., if the identity of the external storage device is verified and approved), the medical device can transition from the first mode of operation to a second mode of operation (e.g., a safe operation mode). During the second mode of operation, some or all device-specific tasks are suspended to ensure that the medical device does not malfunction in the scenario that the external storage device includes malware. As a non-limiting example, if the medical device is a defibrillator, some or all defibrillation operations can be suspended (e.g., disabled) during the second mode of operation. For example, vital sign monitoring on the defibrillator may continue, but shock determinations and charging of therapy electrodes may be suspended. As another non-limiting example, if the medical device is a patient monitoring device, vital sign monitoring and recording can be suspended.

A second authentication check can be performed when the medical device transitions to the second mode of operation. During the second authentication check, the medical device can identify a content manifest associated with the external storage device. The content manifest can identify the content (e.g., files, documents, programs, etc.) that should be stored on the external storage device. In some scenarios, to ensure that the content manifest is authentic, the medical device can search for a digital signature on the content manifest and verify the authenticity of the digital signature. Additionally, during the second authentication check, the medical device can verify that the actual content stored on the external storage device matches the content identified in the content manifest. If the actual content stored on the external storage device matches the content identified in the content manifest, the external storage device passes the second authentication check and the medical device can safely mount the external storage device. Additionally, the medical device can transition back to the first mode of operation.

In some scenarios, an accessory device (e.g., a cardiopulmonary resuscitation (CPR) puck, a vital sensor, a video laryngoscope, an ultrasound probe, etc.) can be inserted (e.g., plugged into) an accessory device interface of the medical device. As used herein, an "accessory device" corresponds to any device that supports, supplements, or augments the performance of the medical device. In one embodiment, the accessory device interface can be the same interface as the external storage device interface. As a non-limiting example, the accessory device interface and the external storage device interface can correspond to a USB interface. Upon detecting the insertion of the accessory device, the medical device can transition to the second mode of operation (e.g., the safe operation mode) and perform an accessory device authentication check while the devices-specific tasks are suspended.

In some embodiments, the accessory device authentication check can be performed by a remote server. For example, to perform the accessory device authentication check, the medical device can identify different attributes of the accessory device and send the attributes to the remote server. Based on the attributes, the remote server can send an indication of whether the accessory device passes the accessory device authentication check.

In other embodiments, the accessory device authentication check can be performed by the medical device. For example, the remote server can send a list of approved accessory devices to the medical device, and the medical device can verify whether the accessory device is on the list of approved accessory devices.

If the accessory device passes the accessory device authentication check, the medical device can transition to the first mode of operation and can utilize the accessory device in conjunction with treating a patient. However, if the accessory device does not pass the authentication check, to reduce the likelihood of malfunction, the medical device can remain in the second mode of operation until the accessory device is removed.

Thus, the techniques described herein provide different authentication techniques for external sources (e.g., storage drives and accessory devices) that are inserted into the medical device. By operating the medical device in the second mode of operation until the external sources are authenticated, the likelihood of an external source with malware causing the medical device to malfunction is greatly reduced.

The figures and the following description illustrate specific exemplary embodiments. It will be appreciated that those skilled in the art will be able to devise various arrangements that, although not explicitly described or shown herein, embody the principles described herein and are included within the scope of the claims that follow this description. Furthermore, any examples described herein are intended to aid in understanding the principles of the disclosure and are to be construed as being without limitation. As a result, this disclosure is not limited to the specific embodiments or examples described below, but by the claims and their equivalents.

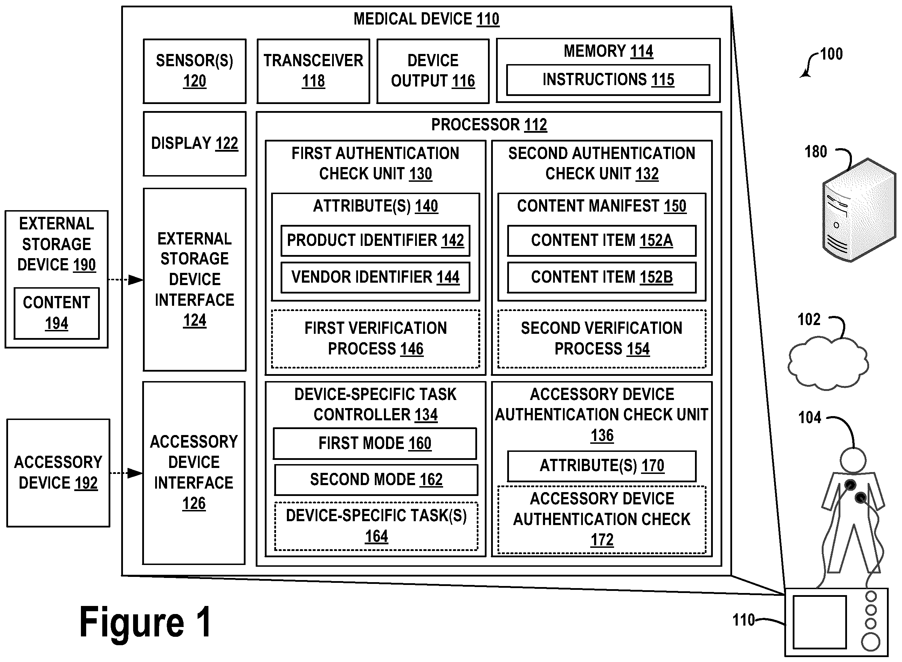

Particular implementations are described herein with reference to the drawings. In the description, common features may be designated by common reference numbers throughout the drawings. In some drawings, multiple instances of a particular type of feature are used. Although these features are physically and/or logically distinct, the same reference number is used for each, and the different instances are distinguished by addition of a letter to the reference number. When the features as a group or a type are referred to herein (e.g., when no particular one of the features is being referenced), the reference number is used without a distinguishing letter. However, when one particular feature of multiple features of the same type is referred to herein, the reference number is used with the distinguishing letter. For example, referring to Figure 1, content items are illustrated and associated with reference number 152. When referring to a particular one of the content items, such as the content item 152A, the distinguishing letter "A" is used. However, when referring to any arbitrary one of the content items or to the content items as a group, the reference number 152 may be used without a distinguishing letter.

As used herein, various terminology is used for the purpose of describing particular implementations only and is not intended to be limiting. For example, the singular forms "a," "an," and "the" are intended to include the plural forms as well, unless the context clearly indicates otherwise. Further, the terms "comprise," "comprises," and "comprising" are used interchangeably with "include," "includes," or "including." Additionally, the term "wherein" is used interchangeably with the term "where." As used herein, "exemplary" indicates an example, an implementation, and/or an aspect, and should not be construed as limiting or as indicating a preference or a preferred implementation. As used herein, an ordinal term (e.g., "first," "second," "third," etc.) used to modify an element, such as a structure, a component, an operation, etc., does not by itself indicate any priority or order of the element with respect to another element, but rather merely distinguishes the element from another element having a same name (but for use of the ordinal term). As used herein, the term "set" refers to a grouping of one or more elements, and the term "plurality" refers to multiple elements.

Referring to Figure 1, a system 100 that is operable to authenticate external devices inserted into a medical device is illustrated, in accordance with an exemplary embodiment. The system 100 includes a medical device 110 and a server 180.

The medical device 110 can communicate with the server 180 using a network 102. For example, the medical device 110 can send data to the server 180 using the network 102, and the server 180 can send data to the medical device using the network 102. According to one implementation, the network 102 is an Institute of Electrical and Electronics Engineers (IEEE) 802.11 (WiFi) network. According to one implementation, the network 102 is a cellular network, such as a public land mobile network (PLMN).

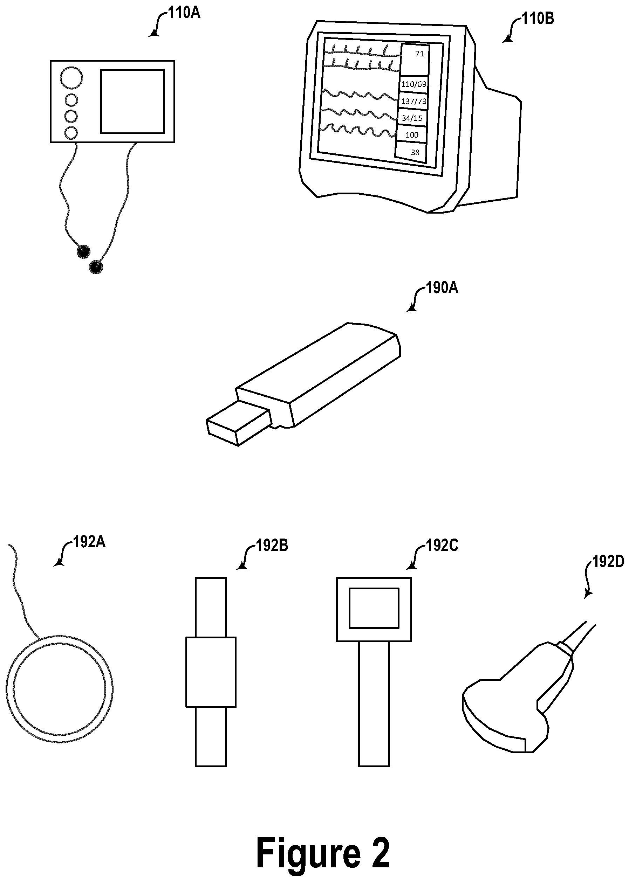

In Figure 1, the medical device 110 is depicted as a defibrillator that is operable to perform defibrillation operations on a patient 104. It should be understood that the defibrillator depicted in Figure 1 is merely one non-limiting example of the medical device 110. In other implementations, as described with respect to Figure 2, the medical device 110 can include a patient monitoring device or any other device that is operable to treat or monitor the patient 104.

The medical device 110 includes a processor 112 and a memory 114 coupled to the processor 112. The memory 114 can correspond to a non-transitory computer-readable medium that includes instructions 115 that are executable by the processor 112 to perform the operations described herein. The medical device 110 also includes a device output 116 coupled to the processor 112, a transceiver 118 coupled to the processor 112, one or more sensors 120 coupled to the processor 112, a display 122 coupled to the processor 112, an external storage device interface 124 coupled to the processor 112, and an accessory device interface 126 coupled to the processor 112. In some implementations, the external storage device interface 124 and the accessory device interface 126 are the same interface. As a non-limiting example, a single USB interface can function as the external storage device interface 124 and/or the accessory device interface 126. It should be understood that the components of the medical device 110 depicted in Figure 1 are for illustrative purposes and should not be construed as limiting. In some implementations, the medical device 110 can include additional or fewer components.

The processor 112 can be configured to authenticate devices, such as an external storage device 190 or an accessory device 192, inserted into the medical device 110 to ensure that the devices 190, 192 do not include malware (e.g., malicious software) that can cause the medical device 110 to malfunction. The processor 112 includes a first authentication check unit 130, a second authentication check unit 132, a device-specific task controller 134, and an accessory device authentication check unit 136. In some implementations, one or more of the components 130, 132, 134, 136 of the processor 112 can be implemented using software. For example, one or more of the components 130, 132, 134, 136 of the processor 112 can be implemented by the processor 112 executing the instructions 115 stored in the memory 114. In some implementations, one or more of the components 130, 132, 134, 136 of the processor 112 can be implemented using dedicated circuity. As a non-limiting example, one or more of the components 130, 132, 134, 136 of the processor 112 can be implemented using one or more application-specific integrated circuits (ASICs) or one or more field-programmable gate array (FPGA) devices.

The device-specific task controller 134 can be configured to operate in a first mode 160 of operation or a second mode 162 of operation. While operating in the first mode 160 of operation, the device-specific task controller 134 can perform one or more device-specific tasks 164. As non-limiting examples, if the medical device 110 is a defibrillator, the device-specific tasks 164 can include defibrillation operations (e.g., applying an electric charge or current to a heart to restore a normal heartbeat). To illustrate, the device output 116 can be a set of defibrillator paddles that are positioned proximate to the heart of the patient 104, and the device-specific task controller 134 can perform the device-specific tasks 164 by applying an electric charge to the defibrillator paddles. As another non-limiting example, if the medical device 110 is a patient monitoring device, the device-specific tasks 164 can include monitoring and recording vital signs of the patient 104. While operating in the second mode 162 of operation, the device-specific tasks 164 are suspended. As described below, during at least part of the process for authenticating the external devices 190, 192, the device-specific task controller 134 transitions to the second mode 162 of operation (e.g., a "safe mode") to ensure there is not a malfunction with the medical device 110 (caused by malware associated with the external devices 190, 192) while performing the device-specific tasks 164.

The processor 112 is configured to detect an insertion of the external storage device 190 into the external storage device interface 124. As described with respect to Figure 2, among other things, the external storage device 190 can be a thumb drive or a USB drive. In response to detecting the insertion of the external storage device 190 into the external storage device interface 124, the first authentication check unit 130 can be configured to perform a first authentication check on the external storage device 190.

To perform the first authentication check on the external storage device 190, the first authentication check unit 130 can be configured to identify one or more attributes 140 of the external storage device 190. The one or more attributes 140 can include a product identifier 142 of the external storage device 190, a vendor identifier 144 of the external storage device 190, or both. The first authentication check unit 130 can be configured to perform a first verification process 146 to verify an identity of the external storage device 190 based on the one or more attributes 140. For example, the first authentication check unit 130 can determine whether the product identifier 142 and/or the vendor identifier 144 belongs to a registered external storage device 190. In some scenarios, the first authentication check unit 130 can send, via the transceiver 118 and the network 102, a request to the server 180 to verify the product identifier 142 and/or the vendor identifier 144. The external storage device 190 passes the first authentication check in response to verification of the identity of the external storage device 190. If the external storage device 190 does not pass the first authentication check (e.g., if the identity of the external storage device 190 is not verified), then the processor 112 can determine not to mount content 194 stored on the external storage device 190.

In response to the external storage device 190 passing the first authentication check, the processor 112 can be configured to transition the device-specific task controller 134 from the first mode 160 of operation to the second mode 162 of operation. As a result, the device-specific task controller 134 suspends the device-specific tasks 164 while operating in the second mode 162 of operation. While the device-specific task controller 134 is operating in the second mode 162 of operation, the second authentication check unit 132 can be configured to perform a second authentication check on the content 194 stored on the external storage device 190.

To perform the second authentication check on the content 194 stored on the external storage device 190, the second authentication check unit 132 can be configured to identify a content manifest 150 associated with the external storage device 190. The content manifest 150 can be located (e.g., stored) on the external storage device 190 or can be accessible to the medical device 110 via the server 180. In some implementations, to increase security and reduce the likelihood of malware, upon identifying the content manifest 150, the second authentication check unit 132 can verify whether the content manifest 150 is digitally signed by an authorized party. In these implementations, if the content manifest 150 is not digitally signed by an authorized party, the processor 112 can bypass the second authentication check and determine not to mount content 194 stored on the external storage device 190.

The content manifest 150 includes a list of content items 152 that should be stored on the external storage device 190. As depicted in Figure 1, the content manifest 150 includes a content item 152A and a content item 152B. It should be understood that in other implementations, the content manifest 150 can include additional or fewer content items 152. As a non-limiting example, in some implementations, the content manifest 150 can include forty (40) content items 152. In other implementations, the content manifest 150 can include a single content item 152. The second authentication check unit 132 can be configured to perform a second verification process 154 to verify the content 194 stored on the external storage device 190 matches the list of content items 152. In some scenarios, the content 194 stored on the external storage device 190 matches the list of content items 152 if each content item 152A, 152B in the list of content items 152 has associated content 194 stored on the external storage device 190. In other scenarios, the content 194 stored on the external storage device 190 matches the list of content items 152 if all of the content 194 stored on the external storage device 190 is included in the list of content items 152. The content 194 stored on the external storage device 190 passes the second authentication check in response to verification that the content 194 stored on the external storage device 190 matches the list of content items 152.

In response to the content 194 stored on the external storage device 190 passing the second authentication check, the processor 112 can be configured to mount the content 194 stored on the external storage device 190. Additionally, in response to the content 194 stored on the external storage device 190 passing the second authentication check, the processor 112 can transition the device-specific task controller 134 from the second mode 162 of operation back to the first mode 160 of operation to enable the device-specific task controller 134 to resume performance of the device-specific tasks 164. However, if the content 194 stored on the external storage device 190 does not pass the second authentication check, the processor 112 can disconnect the external storage device 190 to prevent any potential malware from causing the medical device 110 to malfunction.

The processor 112 can also be configured to detect an insertion of the accessory device 192 into the accessory device interface 126. As described with respect to Figure 2, among other things, the accessory device 192 can be a CPR puck, a vital sensor, a video laryngoscope, or an ultrasound probe. In response to detecting the insertion of the accessory device 192 into the accessory device interface 126, the processor 112 can be configured to transition the device-specific task controller 134 from the first mode 160 of operation to the second mode 162 of operation. Thus, in response to detecting the insertion of the accessory device 192 into the accessory device interface 126, the device-specific tasks 164 are suspended.

While the device-specific task controller 134 is operating in the second mode 162 of operation, the accessory device authentication check unit 136 can be configured to perform an accessory device authentication check 172 on the accessory device 192 based on information from the remote server 180. To illustrate, the accessory device authentication check unit 136 can identify one or more attributes 170 of the accessory device 192. The attributes 170 can include a product identifier of the accessory device 192, a vendor identifier of the accessory device 192, or both.

In one implementation, the accessory device authentication check unit 136 can be configured to send, via the transceiver 118 and the network 102, the attributes 170 of the accessory device 192 to the remote server 180. In response to sending the attributes 170 of the accessory device 192 to the remote server 180, the accessory device authentication check unit 136 can receive, from the server 180, an indication of whether the accessory device 192 passed the accessory device authentication check 172. Thus, in this implementation, the accessory device authentication check 172 is performed by the server 180 and the results of the accessory device authentication check 172 is provided to the medical device 110 (e.g., the accessory device authentication check unit 136).

In another implementation, the accessory device authentication check unit 136 can be configured to verity, based on the attributes 170 of the accessory device 192, whether the accessory device 192 is on a list of approved accessory devices from the remote server 180. Thus, in this implementation, the server 180 provides the medical device 110 with an approved list of accessory devices. The accessory device 192 passes the accessory device authentication check 172 in response to verification that the accessory device 192 is on the list of approved accessory devices.

If the accessory device 192 passes the accessory device authentication check 172, the processor 112 can mount the accessory device 192 for use and transition the device-specific task controller 134 back into the first mode 160 of operation to enable performance of the device-specific tasks 164. However, if the accessory device 192 does not pass the accessory device authentication check 172, the processor 112 can disconnect the accessory device 192 to prevent any potential malware from causing the medical device 110 to malfunction.

The techniques described with respect to Figure 1 provide different authentication techniques for external devices (e.g., the external storage device 190 and the accessory device 192) that are inserted into the medical device 110. By operating the medical device 110 in the second mode 162 of operation until the external devices 190, 192 are authenticated, the likelihood of an external device 190, 192 with malware causing the medical device 110 to malfunction is greatly reduced.

Figure 2 illustrates a different examples of medical devices, external storage devices, and accessory devices, according to an exemplary embodiment. As depicted in Figure 2, the medical device 110 can include a defibrillator 110A or a patient monitoring device 110B. The external storage device 190 can include a thumb drive 190A (e.g., a USB drive). The accessory device 192 can include a CPR puck 192A, a vital sensor 192B, a video laryngoscope 192C, or an ultrasound probe 192D.

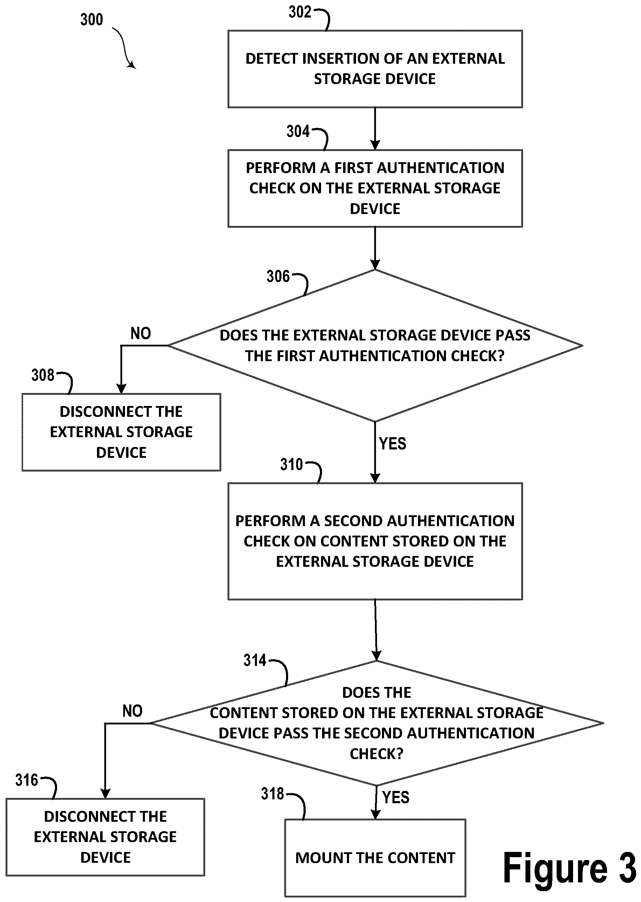

Figure 3 illustrates a process 300 for authenticating an external storage device inserted into a medical device, according to an exemplary embodiment. The process 300 can be performed by the medical device 110 of Figure 1.

According to the process 300, at step 302, the medical device 110 can detect the insertion of the external storage device 190. To illustrate, the processor 112 can detect the insertion of the external storage device 190 in the external storage device interface 124.

At step 304, the medical device 110 can perform a first authentication check on the external storage device. To illustrate, the first authentication check unit 130 can identify the attributes 140 of the external storage device 190 and verify the identity of the external storage device 190 based on the attributes 140. The external storage device 190 passes the first authentication check in response to verification of the identity of the external storage device 190.

At step 306, the medical device 110 can determine whether the external storage device 190 passed the first authentication check. If the medical device 110 determines that the external storage device 190 did not pass the first authentication check, at step 308, the medical device 110 can disconnect the external storage device 190. However, if the medical device 110 determines that the external storage device 190 passed the first authentication check, at step 310, the medical device 110 can transition into a safe operating mode (e.g., the second mode 162 of operation) and perform a second authentication check on the content 194 stored on the external storage device 190.

At step 314, the medical device 110 can determine whether the content 194 stored on the external storage device 190 passes the second authentication check. If the medical device 110 determines that the content 194 stored on the external storage device 190 did not pass the second authentication check, at step 316, the medical device 110 can disconnect the external storage device 190. However, if the medical device 110 determines that the content 194 stored on the external storage device 190 did pass the second authentication check, at step 318, the medical device 110 can mount the content 194.

The process 300 of Figure 3 provides an authentication technique for the external storage device 190 inserted into the medical device 110. By operating the medical device 110 in the second mode 162 of operation until the external storage device 190 is authenticated, the likelihood of an external storage device 190 with malware causing the medical device 110 to malfunction is greatly reduced.

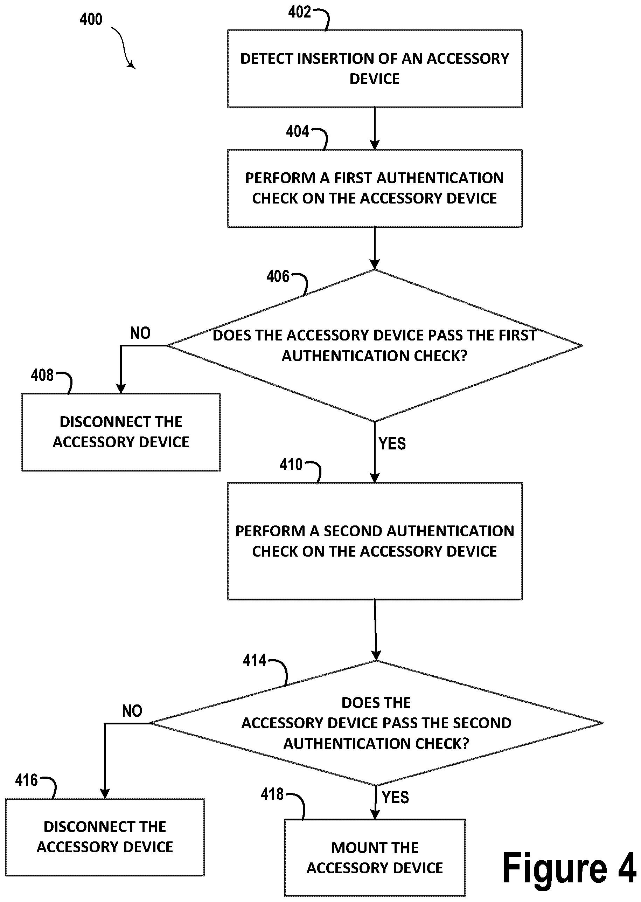

Figure 4 illustrates a process 400 for authenticating an accessory device inserted into a medical device, according to an exemplary embodiment. The process 400 can be performed by the medical device 110 of Figure 1.

According to the process 400, at step 402, the medical device 110 can detect the insertion of the accessory device 192. To illustrate, the processor 112 can detect the insertion of the accessory device 192 in the accessory device interface 126.

At step 404, the medical device 110 can perform a first authentication check on the accessory device 192.

At step 406, the medical device 110 can determine whether the accessory device 192 passed the first authentication check 406. If the accessory device 192 does not pass the first authentication check, the medical device 110 can disconnect the accessory device 192, at step 408.

However, if the accessory device 192 passes the first authentication check, the medical device 110 can perform a second authentication check on the accessory device 192, at step 410.
According to one implementation, the accessory device authentication check 172 is performed by the server 180 and the results of the accessory device authentication check 172 is provided to the medical device 110. According to another implementation, the server 180 provides the medical device 110 with an approved list of accessory devices. The accessory device 192 passes the accessory device authentication check 172 in response to verification that the accessory device 192 is on the list of approved accessory devices.

At step 414, the medical device 110 determines whether the accessory device 192 passed the second authentication check on the accessory device 192. If the accessory device 192 did not pass the second authentication check, at step 416, the medical device 110 disconnects the accessory device 192. However, if the accessory device 192 passed the second authentication check, at step 418, the medical device 110 mounts the accessory device 192.

The process 400 of Figure 4 provides an authentication technique for the accessory device 192 inserted into the medical device 110. By operating the medical device 110 in the second mode 162 of operation until the accessory device 192 is authenticated, the likelihood of an accessory device 192 with malware causing the medical device 110 to malfunction is greatly reduced.

In some implementations, the process 300 and the process 400 can be combined into a single process performed by a medical device, such as the medical device 110 of Figure 1. For example, prior to, during, or after authenticating the external storage device 190 using at least part of the process 300 of Figure 3, the medical device 110 can authenticate the accessory device 192 using at least part of the process 400 of Figure 4.

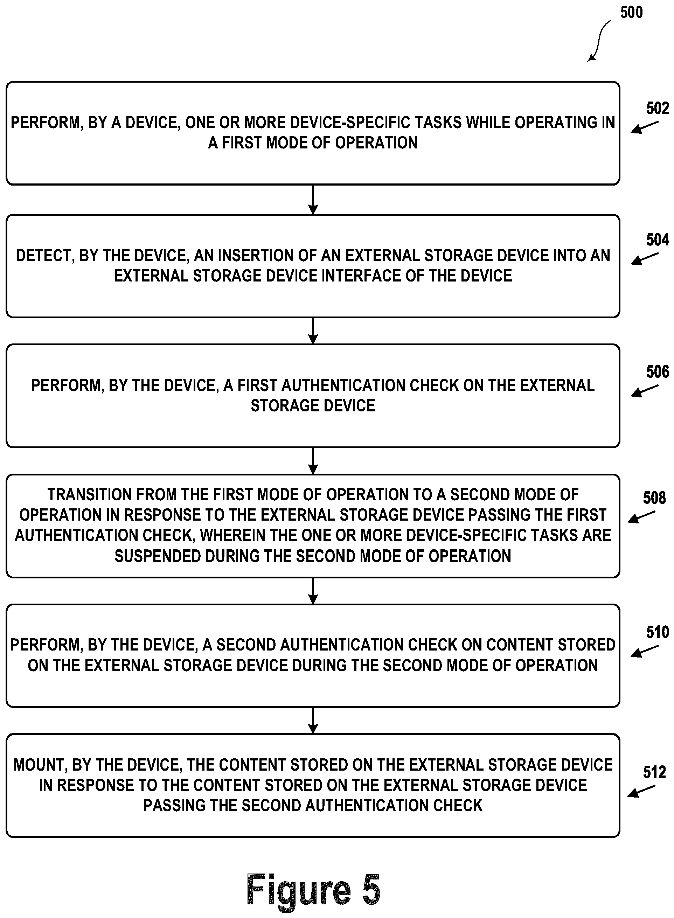

Figure 5 illustrates a flowchart of a method 500 for authenticating external devices inserted into a medical device, according to an exemplary embodiment. The method 500 can be performed by the medical device 110 of Figure 1. According to some implementations of the method 500, the medical device 110 corresponds to a defibrillator or a patient monitor.

The method 500 includes performing, by a device, one or more device-specific tasks while operating in a first mode of operation, at block 502. For example, referring to Figure 1, the device-specific task controller 134 performs the device-specific tasks 164 while operating in the first mode 160 of operation.

The method 500 also includes detecting, by the device, an insertion of an external storage device into an external storage device interface of the device, at block 504. For example, referring to Figure 1, the processor 112 detects the insertion of the external storage device 190 into the external storage device interface 124. According to some implementations, the external storage device 190 corresponds to a thumb drive or a USB drive.

The method 500 also includes performing, by the device, a first authentication check on the external storage device, at block 506. For example, referring to Figure 1, the first authentication check unit 130 performs the first authentication check on the external storage device 190.

According to one implementation of the method 500, performing the first authentication check on the external storage device 190 includes identifying one or more attributes 140 of the external storage device 190. The one or more attributes 140 can include the product identifier 142 of the external storage device 190, the vendor identifier 144 of the external storage device 190, or both. Performing the first authentication check on the external storage device 190 can also include performing the first verification process to verify the identity of the external storage device 190 based on the one or more attributes 140. The external storage device 190 passes the first authentication check in response to verification of the identity of the external storage device 190.

The method 500 also includes transitioning from the first mode of operation to a second mode of operation in response to the external storage device passing the first authentication check, at block 508. The one or more device-specific tasks are suspended during the second mode of operation. For example, referring to Figure 1, the device-specific task controller 134 transitions from the first mode 160 of operation to the second mode 162 of operation in response to the external storage device 190 passing the first authentication check. The device-specific tasks 164 are suspended during the second mode 162 of operation.

The method 500 also includes performing, by the device, a second authentication check on content stored on the external storage device during the second mode of operation, at block 510. For example, referring to Figure 1, the second authentication check unit 132 performs the second authentication check on the content 194 stored on the external storage device 190 during the second mode 162 of operation.

According to one implementation of the method 500, performing the second authentication check on the content 194 stored on the external storage device 190 includes identifying the content manifest 150 associated with the external storage device 190. The content manifest 150 indicates the list of content items 152 that should be stored on the external storage device 190. Performing the second authentication check on the content 194 stored on the external storage device 190 also includes performing the second verification process to verify the content 194 stored on the external storage device 190 matches the list of content items 152. The content 194 stored on the external storage device 190 passes the second authentication check in response to verification that the content 194 stored on the external storage device 190 matches the list of content items 152. According to some implementations, the content 194 stored on the external storage device 190 matches the list of content items 152 if each content item 152 in the list of content items 152 has associated content 194 stored on the external storage device 190. According to other implementations, the content 194 stored on the external storage device 190 matches the list of content items 152 if all of the content 194 stored on the external storage device 190 is included in the list of content items 152. According to some implementations, the content manifest 150 is digitally signed.

The method 500 also includes mounting, by the device, the content stored on the external storage device in response to the content stored on the external storage device passing the second authentication check, at block 512. For example, referring to Figure 1, the processor 112 mounts the content 194 stored on the external storage device 190 in response to the content 194 stored on the external storage device 190 passing the second authentication check.

The method 500 of Figure 5 provides an authentication technique for the external storage device 190 inserted into the medical device 110. By operating the medical device 110 in the second mode 162 of operation until the external storage device 190 is authenticated, the likelihood of an external storage device 190 with malware causing the medical device 110 to malfunction is greatly reduced.

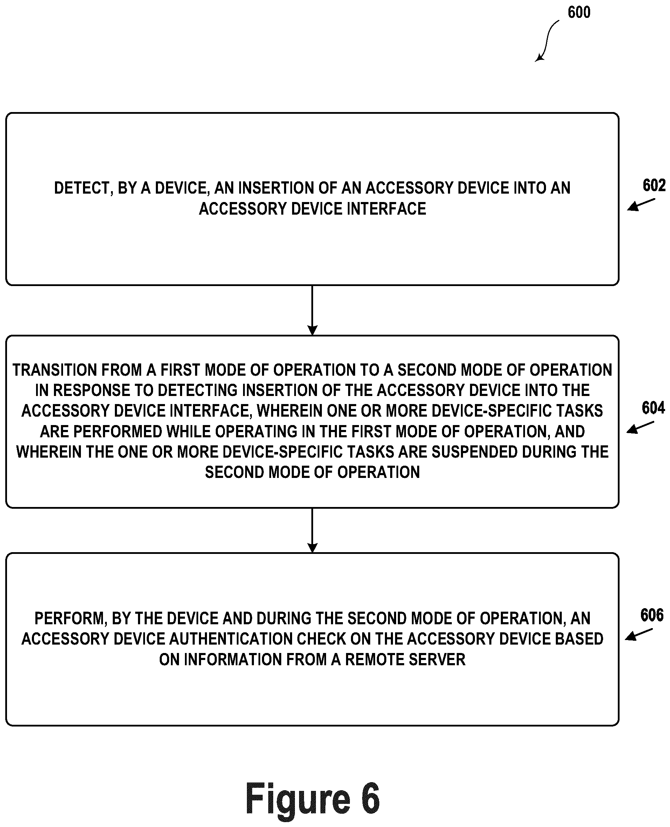

Figure 6 illustrates a flowchart of a method 600 for authenticating external devices inserted into a medical device, according to an exemplary embodiment. The method 600 can be performed by the medical device 110 of Figure 1. According to some implementations of the method 600, the medical device 110 corresponds to a defibrillator or a patient monitor.

The method 600 includes detecting, by a device, an insertion of an accessory device into an accessory device interface, at block 602. For example, referring to Figure 1, the processor 112 detects the insertion of the accessory device 192 into the accessory device interface 126. According to one implementation, the accessory device 192 includes a CPR puck, a vital sensor, a video laryngoscope, or an ultrasound probe.

The method 600 also includes transitioning from a first mode of operation to a second mode of operation in response to detecting insertion of the accessory device into the accessory device interface, at block 604. One or more device-specific tasks are performed while operating in the first mode of operation, and the one or more device-specific tasks are suspended during the second mode of operation. For example, referring to Figure 1, the processor 112 transitions the device-specific task controller 134 from the first mode 160 of operation to the second mode 162 of operation in response to detecting the insertion of the accessory device 192 into the accessory device interface 126. The device-specific tasks 164 are performed while operating in the first mode 160 of operation, and the device-specific tasks 164 are suspended during the second mode 162 of operation.

The method 600 also includes performing, by the device and during the second mode of operation, an accessory device authentication check on the accessory device based on information from a remote server, at block 606. For example, referring to Figure 1, the accessory device authentication check unit 136 performs, during the second mode 162 of operation of the device-specific task controller 134, the accessory device authentication check on the accessory device 192 based on information from the remote server 180.

According to one implementation of the method 600, performing the accessory device authentication check on the accessory device 192 includes identifying one or more attributes 170 of the accessory device 192 and sending the one or more attributes 170 to the remote server 180. Performing the accessory device authentication check on the accessory device 192 also includes receiving, from the remote server 180 and in response to sending the one or more attributes 170 to the remote server 180, an indication of whether the accessory device 192 passed the accessory device authentication check.

According to one implementation of the method 600, performing the accessory device authentication check on the accessory device 192 includes identifying one or more attributes 170 of the accessory device 192. Performing the accessory device authentication check on the accessory device 192 also includes verifying, based on the one or more attributes 170, whether the accessory device 192 is on a list of approved accessory devices from the remote server. The accessory device 192 passes the accessory device authentication check in response to verification that the accessory device 192 is on the list of approved accessory devices.

The method 600 of Figure 6 provides an authentication technique for the accessory device 192 inserted into the medical device 110. By operating the medical device 110 in the second mode 162 of operation until the accessory device 192 is authenticated, the likelihood of an accessory device 192 with malware causing the medical device 110 to malfunction is greatly reduced.

In some implementations, the method 500 and the method 600 can be combined into a single method performed by a medical device, such as the medical device 110 of Figure 1. For example, prior to, during, or after authenticating the external storage device 190 using at least part of the method 500 of Figure 5, the medical device 110 can authenticate the accessory device 192 using at least part of the method 600 of Figure 6.

Additionally, instances in this specification where one element is "coupled" to another element can include direct and indirect coupling. Direct coupling can be defined as one element coupled to and in some contact with another element. Indirect coupling can be defined as coupling between two elements not in direct contact with each other, but having one or more additional elements between the coupled elements. Further, as used herein, securing one element to another element can include direct securing and indirect securing. Additionally, as used herein, "adjacent" does not necessarily denote contact. For example, one element can be adjacent another element without being in contact with that element.

As used herein, a system, apparatus, structure, article, element, component, or hardware "configured to" perform a specified function is indeed capable of performing the specified function without any alteration, rather than merely having potential to perform the specified function after further modification. In other words, the system, apparatus, structure, article, element, component, or hardware "configured to" perform a specified function is specifically selected, created, implemented, utilized, programmed, and/or designed for the purpose of performing the specified function. As used herein, "configured to" denotes existing characteristics of a system, apparatus, structure, article, element, component, or hardware which enable the system, apparatus, structure, article, element, component, or hardware to perform the specified function without further modification. For purposes of this disclosure, a system, apparatus, structure, article, element, component, or hardware described as being "configured to" perform a particular function may additionally or alternatively be described as being "adapted to" and/or as being "operative to" perform that function.

The flow chart diagrams included herein are generally set forth as logical flow chart diagrams. As such, the depicted order and labeled steps are indicative of one embodiment of the presented method. Other steps and methods may be conceived that are equivalent in function, logic, or effect to one or more steps, or portions thereof, of the illustrated method. Additionally, the format and symbols employed are provided to explain the logical steps of the method and are understood not to limit the scope of the method. Although various arrow types and line types may be employed in the flow chart diagrams, they are understood not to limit the scope of the corresponding method. Indeed, some arrows or other connectors may be used to indicate only the logical flow of the method. For instance, an arrow may indicate a waiting or monitoring period of unspecified duration between enumerated steps of the depicted method. Additionally, the order in which a particular method occurs may or may not strictly adhere to the order of the corresponding steps shown.

Unless otherwise indicated, the terms "first," "second," etc. are used herein merely as labels, and are not intended to impose ordinal, positional, or hierarchical requirements on the items to which these terms refer. Moreover, reference to, e.g., a "second" item does not require or preclude the existence of, e.g., a "first" or lower-numbered item, and/or, e.g., a "third" or higher-numbered item.

While the systems and methods of operation have been described with reference to certain examples, it will be understood by those skilled in the art that various changes can be made and equivalents can be substituted without departing from the scope of the claims. Therefore, it is intended that the present methods and systems not be limited to the particular examples disclosed, but that the disclosed methods and systems include all embodiments falling within the scope of the appended claims.

## Claims

1. A device comprising:
an external storage device interface;
a processor; and
a memory storing computer-executable instructions that, when executed by the processor, cause the processor to:
perform one or more device-specific tasks while operating in a first mode of operation;
detect an insertion of an external storage device into the external storage device interface;
perform a first authentication check on the external storage device;
transition from the first mode of operation to a second mode of operation in response to the external storage device passing the first authentication check, wherein the one or more device-specific tasks are suspended during the second mode of operation;
perform a second authentication check on content stored on the external storage device during the second mode of operation; and
mount the content stored on the external storage device in response to the content stored on the external storage device passing the second authentication check.

2. The device of claim 1, wherein, to perform the first authentication check on the external storage device, the computer-executable instructions cause the processor to:
identify one or more attributes of the external storage device; and
perform a first verification process to verify an identity of the external storage device based on the one or more attributes, wherein the external storage device passes the first authentication check in response to verification of the identity of the external storage device.

3. The device of claim 2, wherein the one or more attributes comprise a product identifier of the external storage device, a vendor identifier of the external storage device, or both.

4. The device of claim 1, wherein, to perform the second authentication check on the content stored on the external storage device, the computer-executable instructions cause the processor to:
identify a content manifest associated with the external storage device, the content manifest indicating a list of content items that should be stored on the external storage device;
perform a second verification process to verify the content stored on the external storage device matches the list of content items, wherein the content stored on the external storage device passes the second authentication check in response to verification that the content stored on the external storage device matches the list of content items.

5. The device of claim 4, wherein the content stored on the external storage device matches the list of content items if each content item in the list of content items has associated content stored on the external storage device,
wherein optionally the content stored on the external storage device matches the list of content items if all of the content stored on the external storage device is included in the list of content items, or
wherein optionally the content manifest is digitally signed.

6. The device of claim 1, wherein the external storage device interface, the processor, and the memory are integrated into a medical device,
wherein optionally the medical device corresponds to a defibrillator or a patient monitor, or
wherein the external storage device corresponds to a thumb drive or a universal serial bus (USB) drive.

7. The device of claim 1, further comprising an accessory device interface, wherein the computer-executable instructions further cause the processor to:
detect an insertion of an accessory device into the accessory device interface;
transition from the first mode of operation to the second mode of operation in response to detecting insertion of the accessory device into the accessory device interface;
perform, during the second mode of operation, an accessory device authentication check on the accessory device based on information from a remote server,
wherein optionally the accessory device comprises a cardiopulmonary resuscitation (CPR) puck, a vital sensor, a video laryngoscope, or an ultrasound probe.

8. The device of claim 7, wherein, to perform the accessory device authentication check on the accessory device, the computer-executable instructions cause the processor to:
identify one or more attributes of the accessory device; and
send the one or more attributes to the remote server,
receive, from the remote server and in response to sending the one or more attributes to the remote server, an indication of whether the accessory device passed the accessory device authentication check.

9. The device of claim 7, wherein, to perform the accessory device authentication check on the accessory device, the computer-executable instructions cause the processor to:
identify one or more attributes of the accessory device; and
verify, based on the one or more attributes, whether the accessory device is on a list of approved accessory devices from the remote server,
wherein the accessory device passes the accessory device authentication check in response to verification that the accessory device is on the list of approved accessory devices.

10. A method comprising:
performing, by a device, one or more device-specific tasks while operating in a first mode of operation;
detecting, by the device, an insertion of an external storage device into an external storage device interface of the device;
performing, by the device, a first authentication check on the external storage device;
transitioning from the first mode of operation to a second mode of operation in response to the external storage device passing the first authentication check, wherein the one or more device-specific tasks are suspended during the second mode of operation;
performing, by the device, a second authentication check on content stored on the external storage device during the second mode of operation; and
mounting, by the device, the content stored on the external storage device in response to the content stored on the external storage device passing the second authentication check.

11. The method of claim 10, wherein performing the first authentication check on the external storage device comprises:
identifying one or more attributes of the external storage device; and
performing a first verification process to verify an identity of the external storage device based on the one or more attributes, wherein the external storage device passes the first authentication check in response to verification of the identity of the external storage device.

12. The method of claim 10, wherein performing the second authentication check on the content stored on the external storage device comprises
identifying a content manifest associated with the external storage device, the content manifest indicating a list of content items that should be stored on the external storage device;
performing a second verification process to verify the content stored on the external storage device matches the list of content items, wherein the content stored on the external storage device passes the second authentication check in response to verification that the content stored on the external storage device matches the list of content items.

13. The method of claim 10, further comprising:
detecting, by the device, an insertion of an accessory device into an accessory device interface;
transitioning from the first mode of operation to the second mode of operation in response to detecting insertion of the accessory device into the accessory device interface;
performing, by the device and during the second mode of operation, an accessory device authentication check on the accessory device based on information from a remote server.

14. The method of claim 13, wherein performing the accessory device authentication check on the accessory device comprises:
identifying one or more attributes of the accessory device; and
sending the one or more attributes to the remote server,
receiving, from the remote server and in response to sending the one or more attributes to the remote server, an indication of whether the accessory device passed the accessory device authentication check.

15. A non-transitory computer-readable medium comprising instructions that, when executed by a processor of a device, causes the processor to perform operations comprising:
performing one or more device-specific tasks while operating in a first mode of operation;
detecting an insertion of an external storage device into an external storage device interface of the device;
performing a first authentication check on the external storage device;
transitioning from the first mode of operation to a second mode of operation in response to the external storage device passing the first authentication check, wherein the one or more device-specific tasks are suspended during the second mode of operation;
performing a second authentication check on content stored on the external storage device during the second mode of operation; and
mounting the content stored on the external storage device in response to the content stored on the external storage device passing the second authentication check.
